# EUROPEAN PATENT APPLICATION

(11) **EP 1 698 359 A1**
(43) Date of publication of application: **06.09.2006**
(21) Application number: 04820674.2
(22) Date of filing: 07.12.2004
(51) Int. Cl.: A61L 29/06, B29C 41/14, B32B 1/08

(54) **MEDICAL TUBE AND PROCESS FOR PRODUCING THE SAME**

(30) Priority: 22.12.2003 JP 2003425623
(71) Applicant: I.S.T. Corporation, Otsu-shi, Shiga 520-2153 (JP)
(72) Inventor: YAMADA, Hiroshi c/o I.S.T Corporation, Ostu-shi, Shiga 520-2153 (JP); AOYAMA, Chisaka c/o I.S.T Corporation, Ostu-shi, Shiga 520-2153 (JP)
(74) Representative: Schwarzensteiner, Marie-Luise
(86) International application number: PCT/JP2004/018178
(87) International publication number: WO 2005/061020

(57) **Abstract**

A medical tube includes a mixture component including a polyimide resin and a fluorine resin, the mixture component being heated and cured. The fluorine resin melts and is precipitated on at least an inner face of the tube, and the inner face or the inner and outer faces of the tube is a low friction resistance face. This tube is obtained by polymerizing aromatic tetracarboxylic acid dehydrate and aromatic diamine in a polar solvent to be a polyimide precursor solution; adding a fluorine resin in the polyimide precursor solution or during the polymerizing step to prepare a mixed solution of the polyimide precursor and the fluorine resin; applying the mixed solution to an outer face of a core wire so as to have a predetermined thickness; applying heat so as to allow conversion to an imide, where a highest temperature for the conversion to an imide is a temperature exceeding a melting point of the fluorine resin; and cooling it and separating the core wire and the medical tube. Thereby, a medical tube can be provided with a small outer diameter and a small wall thickness and have excellent mechanical characteristics. The inner or the inner and the outer faces of the tube have a low friction resistance. A method for manufacturing the same also can be provided.

## Description

### Technical Field

The present invention relates to a medical tube having excellent mechanical characteristics and being suitable for medical use such as a catheter, made of a mixture containing a polyimide resin as a main ingredient and a fluorine resin. On the inner face or the inner and outer faces of the tube the fluorine resin is precipitated to achieve a low friction resistance. The present invention also relates to a method for manufacturing such a medical tube.

### Background Art

Polyimide resins are excellent in heat resistance, dimensional stability, mechanical characteristics and chemical characteristics, and therefore they are applied to film-form, tube-form or rod-form molded articles to take full advantage of their characteristics. They are commercially available as a varnish and a coating in the form of a polyimide precursor solution, for example, which are applied for various uses such as a flexible printed board, a fixing tube for office automation equipment and an insulation material for heat resistance wires. Especially, it is expected that a relatively thin tube having an inner diameter of 1 mm or less will be used as a tube for terminal insulation tube used with an IC board as well as in the field of a medical tube such as a catheter or an endoscope.

A medical tube such as a catheter is described below. In recent years, along with the highly advanced medical technology, a medical tube also has been required to have advanced functions. That is, due to the fruit of the research and development of medical technology, a catheter treatment and a diagnosis with an endoscope have become dominant instead of a conventional surgical operation in order to alleviate patient's physical and mental suffering and anxiety. In the catheter treatment, a medical instrument such as a catheter is inserted directly into a body cavity of a patient for treatment, and in the endoscope diagnosis, the diseased part is observed for diagnosis using an imaging diagnostic apparatus. A medical tube is used as a catheter and an endoscope in such a new medical technology enabling the above-described catheter treatment and the observation and diagnosis of the diseased part using an imaging diagnostic apparatus.

A medical tube such as a catheter is guided by a guide wire to a diseased part through a minute blood vessel that is arranged complicatedly in a human body. Since such a medical tube has to arrive at the diseased part quickly and accurately, a high operability and a sufficient strength are required. For that reason, a medical tube needs to have the following characteristics: a pushability that is a capability to be pushed into a blood vessel easily; a torque transmission property allowing a torque generated by the operation of a base end portion of the catheter to be transmitted to a tip end portion securely (torque transmission capability), a flexibility that is for avoiding the damage of a blood vessel by the tip end of the catheter; a kink resistant property and the like. Further, in order to alleviate the patient's physical and mental suffering and anxiety, an important property of a medical tube is to have a smallest possible diameter and a thinner wall enabling a larger bore.

In addition to mechanical characteristics including the above-stated torque transmission capability and kink resistant property, such a catheter is required to have a low friction property for the inner and the outer wall faces of the tube. That is, in the catheter treatment, a catheter used for treatment, inserted into a guiding catheter that guides the treatment catheter to a diseased part, is guided by a guide wire made of a metal or the like to the diseased part. In this operation, the outer surface of the catheter for treatment is brought into contact with the inner wall of the guiding catheter and the inner surface is brought into contact with the outer face of the guide wire, and therefore the low friction property of the inner and outer surfaces of the catheter tube also becomes important.

As a method for reducing a friction resistance of the inner and outer faces of the medical tube, the following methods are commonly used. For instance, it includes a method of coating the outer face of a tube with a material having a low friction coefficient such as a fluorine resin and a silicon resin or a method of manufacturing a tube made of a fluorine resin or silicon resin alone and then coating the outer surface of the tube with polyethylene and polyurethane by a multiple extrusion-coating method, so as to enable a small friction resistance of the inner face of the tube.

Most of the currently available catheter tubes are made of many types of materials such as polyurethane, polyethylene, fluorine resin and nylon, and have a composite structure (a tube including a multilayer such as an inner layer, an intermediate layer, an outer layer and the like).

However, according to the above-stated conventional method, an outer diameter and a wall thickness of the tube will be increased. Thus, it is difficult to provide a tube with a smallest possible diameter, a thinner wall and a larger bore, which are required for a medical tube, and to reduce the friction resistance of the outer and inner faces of the tube by a method at a low cost. No materials and manufacturing methods satisfying the above-stated characteristics required for a medical tube such as a catheter have been provided so far, and the research and development of them has been desired.
Patent document 1: JP H07(1995)-51376 A
Patent document 2: JP H10(1998)-314312 A

### Disclosure of Invention

In light of the above-stated conventional problems, it is an object of the present invention to provide a medical tube with a small outer diameter and a small wall thickness and having excellent mechanical characteristics and a low friction resistance of the inner or the inner and the outer faces of the tube, and to provide a method for manufacturing the same.

In order to attain the above-mentioned object, a medical tube of the present invention includes a mixture component including a polyimide resin and a fluorine resin, the mixture component being heated and cured. The fluorine resin melts and is precipitated on an inner face or the inner face and an outer face of the tube, and the face on which the fluorine resin is precipitated is a low friction resistance face.

A method for manufacturing a medical tube of the present invention includes the steps of: polymerizing aromatic tetracarboxylic acid dehydrate and aromatic diamine in a polar solvent to be a polyimide precursor solution; adding a fluorine resin in the polyimide precursor solution or during the polymerizing step to prepare a mixed solution of the polyimide precursor and the fluorine resin; applying the mixed solution to an outer face of a core wire so as to have a predetermined thickness; applying heat so as to allow conversion to an imide, where a highest temperature for the conversion to an imide is a temperature exceeding a melting point of the fluorine resin; and thereafter, separating the core wire and the medical tube.

### Brief Description of Drawings

Fig. 1 is a drawing for explaining a method for measuring a dynamic friction resistance of a medical tube that is used in Examples of the present invention.
Fig. 2A schematically shows the enlarged cross section of a medical tube before the completion of imidization in one example of the present invention.
Fig. 2B schematically shows the enlarged cross section of a medical tube after the completion of imidization in one example of the present invention.

### Description of the Invention

The inventors of the present invention found the following: that is, using a polyimide resin obtained by conversion to an imide by heating of a polyimide precursor solution including at least one type of aromatic tetracarboxylic acid dehydrate and at least one type of aromatic diamine, a fluorine resin such as PTFE, PFA, FEP or PCTFE further is added to such a polyimide precursor solution to prepare a fluorine resin mixed polyimide precursor solution. This solution is molded into a predetermined tube shape, followed by conversion to an imide at a temperature at least exceeding a melting point of the fluorine resin, whereby the fluorine resin melts and is precipitated on the inner face or the inner and outer faces of the tube. Thus, a medical tube with a dynamic friction resistance of the inner face of the tube being 70% or less of that of a tube made of a polyimide resin alone can be obtained. Furthermore, before the conversion to an imide or after completion of the conversion to an imide, a solution containing a polyimide precursor alone is applied again, followed by heating for conversion to an imide, whereby a medical tube having a low friction resistance on the inner face only can be manufactured. The fluorine resin preferably is at least one selected from the group consisting of polytetrafluoroethylene (PTFE), tetrafluoroethylene-perfluoroalkylvinylether copolymer (PFA), polychlorotrifluoroethylene (PCTFE), tetrafluoroethylene-hexafluoropropylene copolymer (FEP) and tetrafluoroethylene-ethylene copolymer (PETFE).

The polyimide resin used in the present invention is a thermosetting resin, and a molded article of the polyimide resin is obtained generally by molding the polyimide precursor solution into a desired shape by casting, flow casting, impregnation or the like, followed by heat treatment for conversion to an imide. In such a method, the mixed solution of a polyimide precursor solution and a fluorine resin of the present invention is allowed to flow over a glass plate or a metal board, which is subjected to casting molding, followed by drying and heating to complete the conversion to an imide. Thereby, a polyimide·fluorine resin composite film, on one side or both sides of which a fluorine resin is precipitated, can be formed.

In the polyimide·fluorine resin composite film or tube manufactured by the above-stated method, the fluorine resin is precipitated on a face of the film that contacts with the air. That is, although the fluorine resin powder mixed in the polyimide precursor solution is present in a uniformly dispersed state, during the procedure of the casting molding of the precursor solution and heating for conversion to an imide, a polymerized solvent and condensed water in the polyimide precursor solution evaporate at the initial heating stage, and the temperature further is increased to a predetermined temperature to complete the imidization. It can be considered that during such a procedure of imidization, the melting fluorine resin moves toward the outermost layer contacting with the air in the thickness direction of the film or the tube.

The details of the phenomenon of the fluorine resin moving in the molded article during the heating of the polyimide precursor for imidization have not been clarified. However, as a result of numerous experiments and researches continued by the inventors of the present invention, it has been found that a fluorine resin can be precipitated also on a face of the film contacting with glass or metal when the above-stated polyimide·fluorine composite film is manufactured, and confirmation experiments were conducted using the medical tube of the present invention.

As a result, it has been found that a fluorine resin can be precipitated also on an inner face of the tube that does not contact with the air layer at all. It has been found further that the phenomenon of the precipitation of the fluorine rein on both faces of the medical tube would be varied depending on a difference of fluorine resin types and a difference of the final temperature for the conversion to an imide.

That is, the phenomenon of the precipitation of a fluorine resin is affected by the melting point of the fluorine resin and the imidization temperature of the polyimide precursor. According to the detailed experimental results, when a PTFE resin (melting point: 327°C) was mixed in a rigid polyimide resin using biphenyl tetracarboxylic acid dehydrate (BPDA) as aromatic tetracarboxylic acid dehydrate and para-phenylenediamine (PPD) as aromatic diamine, the fluorine resin did not appear prominently on either face of the tube at the highest temperature for imidization of 300°C. However, when the temperature was increased to 400°C, the fluorine resin melted and was precipitated on both surfaces of the inner and outer faces of the tube, whereby a tube with a low friction resistance could be obtained.

As a result of the experiment with the polyimide precursor solution using FEP (melting point: 250°C) that was lower than the melting point of PTFE (melting point: 327°C), a fluorine resin (FEP) was precipitated on the inner and outer faces of the tube at the highest temperature for the conversion to an imide of 300°C, whereby a medical tube with a low friction resistance could be obtained.

In this way, concerning the phenomenon of a fluorine resin being precipitated on the inner and outer faces of the medical tube of the present invention, when the melting point of the fluorine resin, the imidization temperature of the polyimide precursor solution and the like are selected and are set at predetermined conditions, the fluorine resin can be precipitated on the both faces of the tube. Therefore, even in the case of a thin tube with an inner diameter of 1 mm or less, a tube having a low friction resistance on the inner and outer faces and suitable for medical use could be obtained. Needless to say, the present invention is applicable also to a tube with an inner diameter exceeding 1 mm.

When the properties of flexibility and stability are to be added by coating the outer face of the medical tube of the present invention with a polyurethane resin or the like, the adhesion between the polyurethane and the polyimide can be enhanced more by using a medical tube without the fluorine resin being precipitated on the outer face. In this way, in order for the tube of the present invention to be ready for combination with a polyurethane resin or the like, before the conversion to an imide or after the completion of the imidization, a solution containing the polyimide precursor alone may be applied again to the medical tube of the present invention as a base, followed by heating for the conversion to an imide, whereby a medical tube having a low friction resistance on the inner face only can be manufactured.

As a material (a monomer of the precursor) to be used for manufacturing a medical tube by applying the solution containing the polyimide precursor alone again as described above, the following are preferable. When more rigidity is required for a tube, the combination of monomers having high mechanical characteristics, such as biphenyl tetracarboxylic acid dehydrate and para-phenylenediamine, may be added to the above-stated materials for the two layers including the layer containing the fluorine resin and the polyimide single layer. When flexibility is required, a precursor of a flexible polyimide resin such as the combination of pyromellitic acid dehydrate and 4,4'- diaminodiphenyl ether may be used.

During the above-stated manufacturing method, before the conversion to an imide or after completion of the imidization, a solution containing the polyimide precursor alone may be applied again, followed by heating for conversion to an imide, and thereafter the core wire and the tube may be separated.

The following describes embodiments of the present invention.

In the present invention, as the fluorine resin, a fluorine resin such as PTFE, PFA, FEP and CPTFE alone or the mixture of them may be used. Since PTFE, PFA and FEP are excellent in heat resistance and mold releasability and are chemically inert, they are excellent for medical use, and these materials preferably are used for the present invention. The amount of the fluorine resin mixed preferably is set at 3 to 50 weight% with reference to a solid content of a polyimide precursor solution. More preferable amount is 10 to 40 weight%. If the content of the above-stated fluorine resin is less than 3 weight%, the effect of reducing a friction resistance is degraded, while if the content exceeds 50 weight%, the mechanical strength is lowered, and the smoothness of the tube surface is impaired and cracks are likely to occur.

The fluorine resin preferably is in the powder form, because it is mixed easily, and the average particle diameter thereof preferably ranges from 0.1 to 25 µm. More preferably, the average particle diameter ranges from 1.0 to 15 µm. Such a range is preferable because the particles can be dispersed uniformly with less flocculation of them. If the average particle diameter is less than 0.1 µm, the particles tend to generate secondary flocculation, while if it exceeds 25 µm, unevenness resulting from the fluorine resin particles tends to occur at the inner face or the outer face of the tube. Thus, such a range is not preferable. Incidentally, the average particle diameter of the above-stated fluorine resin powder can be measured using a laser diffraction particle size analyzer (SALD-2100, produced by Shimadzu Corporation) or a laser scattering particle size distribution analyzer (LA-920, produced by Horiba Ltd.).

The medical tube of the present invention contains a polyimide resin as a main ingredient. More specifically, a fluorine resin and a polyimide precursor solution are mixed, which is subjected to casting molding into a tube shape, followed by heating for converting to an imide. The polyimide precursor solution can be obtained by allowing aromatic tetracarboxylic acid dehydrate and aromatic diamine, which are substantially equimolar, to react in an organic polar solvent.

Typical examples of the aromatic tetracarboxylic acid dehydrate include 3,3',4,4'-benzophenone tetracarboxylic acid dehydrate, pyromellitic acid dehydrate, 2,3,3',4-biphenyl tetracarboxylic acid dehydrate, 3,3'4,4'-biphenyl tetracarboxylic acid dehydrate, 1,2,5,6-naphthalene tetracarboxylic acid dehydrate, 1,4,5,8-naphthalene tetracarboxylic acid dehydrate, 2,3,6,7- naphthalene tetracarboxylic acid dehydrate, 2,2'-bis(3,4-dicarboxyphenyl) propane dehydrate, perylene-3,4,9,10-tetracarboxylic acid dehydrate, bis(3,4-dicarboxyphenyl) ether dehydrate, bis(3,4-dicarboxyphenyl)sulfone dehydrate and the like.

Typical examples of the aromatic diamine include 4,4'-diaminodiphenyl ether, p-phenylenediamine, m-phenylenediamine, 1,5-diamino naphthalene, 3,3'-dichloro benzidine, 3,3'-diamino diphenylmethane, 4,4'-diamino diphenylmethane, 3,3'-dimethyl-4,4'-biphenyl diamine, 4,4'-diamino diphenylsulfide-3,3'-diamino diphenyl sulfone, benzidine, 3,3'-dimethyl benzidine, 4,4'-diamino phenyl sulfone, 4,4'-diamino diphenyl propane, m-xylylene diamine, hexamethylene diamine, diamino propyltetra methylene, 3-methyl heptamethylene diamine and the like.

These aromatic tetracarboxylic acid dehydrates and aromatic diamines may be used alone or in combination of them. Further, the polyimide precursor solution therefor is completed, and these precursors may be mixed for use.

The organic polar solvent, in which the aromatic tetracarboxylic acid dehydrate and the aromatic diamine are allowed to react, includes, for example, N-methyl-2-pyrrolidone, N, N-dimethyl formamide, N, N-dimethyl acetamide, N, N-diethyl formamide, N, N-diethyl acetamide, dimethyl sulfoxide, hexamethylphosphor triamide, pyridine, dimethyl tetramethylene sulfone, tetramethylene sulfone and the like. These organic polar solvents may be mixed with phenol, xylene, hexane, toluene or the like.

The above-mentioned polyimide precursor solution can be obtained by allowing the aromatic tetracarboxylic acid dehydrate and the aromatic diamine to react in an organic polar solvent normally at 90°C or lower, and the solid content in the solvent may be 10 to 30 weight%, which can be set depending on the specifications of the finished polyimide resin tube and the processing conditions.

When the aromatic tetracarboxylic acid dehydrate and the aromatic diamine are allowed to react in the organic polar solvent, the viscosity of the solution may be increased depending on the polymerization conditions. However, this can be diluted to a predetermined viscosity before use. The solution usually is used at the viscosity of 1 to 5,000 poise depending on the manufacturing conditions and working conditions.

Furthermore, an agent for improving mechanical characteristics, such as silica, and contrast agent powder for identifying the state of a catheter tube inserted into a human body by the irradiation with X-ray, such as barium sulfate, may be added to the polyimide precursor·fluorine resin mixed solution of the present invention.

In the manufacturing method of the present invention, in order to achieve a temperature for allowing the conversion to an imide that enables a fluorine resin layer to be precipitated at least on the inner face of the medical tube, heat should be applied to be at a temperature exceeding the melting point of the fluorine resin. The highest temperature for the conversion to an imide preferably is a temperature higher than the melting point of the fluorine resin mixed by 10°C or more, and at this temperature, the conversion to an imide preferably is completed.

A heating time required for precipitating the fluorine resin on the inner and outer faces of the medical tube preferably is within 30 minutes after the highest temperature for the conversion to an imide reaches a temperature exceeding the melding point of the fluorine resin. If heating is conducted for 30 minutes or longer, there is a risk of the thermal decomposition of the fluorine resin and the degradation of the mechanical characteristics of the polyimide resin.

The medical tube of the present invention can be obtained by the following method, for example. The surface of a metal core wire with a predetermined outer diameter (corresponding to the inner diameter of the tube) is covered with a light coating of a fluorine resin mixed polyimide precursor solution via a die, which is introduced into a furnace for imidization, where the polymerized solvent is dried at a relatively low temperature of 100 to 150°C, so as to complete the initial imidization. Such a process is repeated until a predetermined thickness of the tube can be achieved, and after that heat is applied thereto at a temperature exceeding the melting point of the fluorine resin for a predetermined time period as the final imidization condition, whereby the imidization is completed. Thereafter, the core wire is pulled out, so that a tube can be obtained. In the manufacturing of the medical tube of the present invention, since a fluorine resin is precipitated on the inner face of the tube, the core wire can be pulled out easily from the polyimide coated wire obtained by the above-stated method.

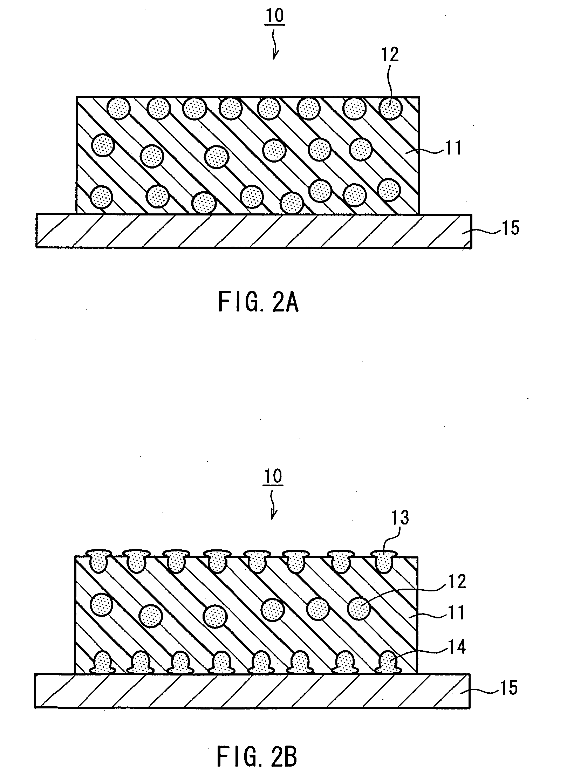

Fig. 2A schematically shows the enlarged cross section of a medical tube 10 according to one embodiment of the present invention when the temperature for imidization is 300°C. Up to this stage, fluorine resin particles are dispersed inside a polyimide film layer 11, and most of the surface layer is covered with a polyimide layer. The contact angle with water also is small at this stage. Reference numeral 15 denotes a core wire.

Next, when the temperature for imidization is increased at 400°C, as shown in Fig. 2B, the fluorine resin melts and is precipitated on the surface layer on the air side and the surface on the base material side so as to seep out from the polyimide resin surface. Reference numeral 10 denotes a partial cross section of the medical tube, 11 denotes a polyimide film layer, 12 denotes internal fluorine resin particles, 13 denotes fluorine resin melting and being precipitated on the outer surface side and 14 denotes fluorine resin melting and being precipitated on the inner surface side. In this state, the contact angle with water becomes high. The fluorine resin does not have compatibility with the polyimide resin, and has a sea-island structure (polyimide resin forms sea and fluorine resin forms island), and the fluorine resin is precipitated partially from the surface of the polyimide resin.

A polyimide film and a sheet form molded article, on one side or both sides of which a fluorine resin is precipitated, can be obtained by letting the fluorine resin mixed polyimide precursor solution flow over the surface of a glass plate or a metal board, which is subjected to casting molding, followed by the completion of the imidization conducted step by step.

The medical tube of the present invention is formed so that a fluorine resin is precipitated on the inner face or the inner and outer faces of the polyimide resin. Since the fluorine resin melts and is precipitated on the inner face or the inner and outer faces of the tube, the faces can have a low friction resistance. Thereby, a medical tube that is substantially a single layer and has a small outer diameter, a small wall thickness and excellent mechanical characteristics, and the inner face or the inner and outer faces thereof have a low friction resistance can be provided, and a method for manufacturing the same can be provided. Further, since the fluorine resin melts and is precipitated on the inner face of the tube, a guide wire and the like can be taken in and out smoothly. Additionally, since the fluorine resin can melt and be precipitated on the outer face also, the occurrence of a thrombus also can be avoided. When the properties of flexibility and stability are to be added by coating the outer face of the medical tube with a polyurethane resin or the like, the adhesion between the polyurethane resin and the polyimide resin can be enhanced more by using a medical tube without the fluorine resin being precipitated on the outer face. In the case of such a composite tube, a medical tube having a low friction resistance on the inner face only can be manufactured at a low cost by applying again a solution containing the polyimide precursor alone before the conversion to an imide or after the completion of the imidization, followed by heating for the conversion to an imide.

### Examples

The following describes the present invention more specifically by way of examples. In the following examples and comparative examples, biphenyl tetracarboxylic acid dehydrate is abbreviated as "BPDA", para-phenylenediamine is abbreviated as "PPD", pyromellitic acid dehydrate is abbreviated as "PMDA", 4,4'-diaminodiphenyl ether is abbreviated as "ODA" and N-methyl-2-pyrrolidone is abbreviated as "NMP".

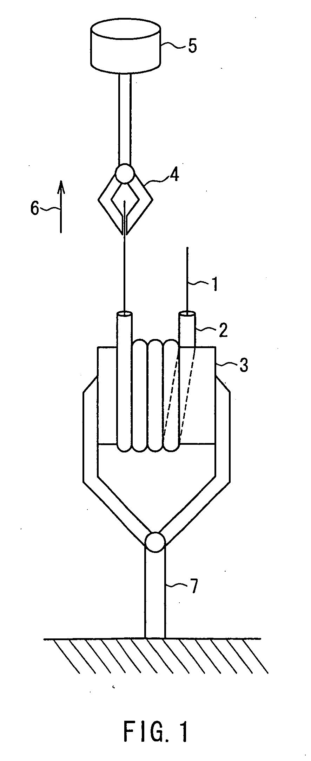

A dynamic friction resistance of a medical tube obtained by the present invention, and a contact angle with pure water and a dynamic friction coefficient of a film obtained from these materials were measured by the following methods.
(1) Method for measuring dynamic friction resistance of medical tube
   As shown in Fig. 1, a nylon wire 1 with a diameter of 0.33 mm and a length of 1,100 mm (produced by Unitika Ltd., "gu-n-ta̅ No. 4"(trade name)) was inserted beforehand into a medical tube 2 with a length of 900 mm. This tube was wound four times around a tube support member 3 made of metal with a diameter of 67 mm. Both end portions of the tube with a length of about 25 mm were held perpendicularly and were fixed with sticky tape, and this support member was attached to a fixing chuck 7 of a tensile tester. After that, one end portion of the nylon wire 1 was fixed with a clip 4 of the tensile tester 5, and was pulled in the direction of an arrow 6 at a rate of 200 mm/minute. Aload at this time was measured, whereby a friction resistance between the inner wall of the medical tube and the outer face of the nylon wire was measured. A smaller tensile load means a smaller friction resistance. During the measurement, values of the tensile load were recorded on a chart across 100 mm of the tensile distance, and the average value was calculated from such data by conducting the test three times.
(2) Measurement of contact angle
   A contact angle with pure water at 23°C was measured using an instrument "FACE CA-Z" produced by Kyowa Interface Science Co., Ltd.

(Example 1)
(1) Manufacturing of fluorine resin mixed polyimide precursor solution
   39 parts by weight of PPD was dissolved in NMP in a flask with respect to 100 parts by weight of BPDA (monomer concentration: 18.2 weight%), which were allowed to react at a temperature of 23°C for 6 hours while stirring, whereby a polyimide precursor solution was obtained. The rotational viscosity of this polyimide precursor solution was 50 poise. Herein, the rotational viscosity was a value measured using a Brookfield viscometer at a temperature of 23°C. Next, PTFE powder with an average particle diameter of 3.0 µm (melting point: 327°C, produced by Dupont, "Zonyl MP1200" (Trade Name)) was added to the polyimide precursor solution so that the ratio became 30 parts by weight of the PTFE powder to 100 parts by weight of solid content in the polyimide precursor solution, followed by stirring so as to disperse the powder uniformly. Subsequently, coarse foreign particles were filtered out using a stainless-steel wire netting of 250 mesh, whereby a fluorine resin powder mixed polyimide precursor solution was prepared.
(2) Manufacturing of medical tube
   Using the fluorine resin mixed polyimide precursor solution prepared by the above-stated process, such a precursor was attached to the surface of a stainless-steel wire with an outer diameter of 0.55 mm and a length of 2,000 mm, which was then allowed to pass through a die with an inner diameter of 0.65 mm so that the precursor could be applied uniformly with a thickness of 7 to 8 µm. Then, this was heated in a furnace for imidization at 100°C for 10 minutes and taken out for the initial imidization processing.

After that, the polyimide precursor solution further was applied thereto, and this was allowed to pass through a die with an inner diameter of 0.75 mm, followed by heating in the furnace for imidization at 100°C for 10 minutes in the same manner as in the above process and cooling again to a room temperature. In this way, the polyimide precursor solution was applied to the outer face of the stainless-steel wire with an outer diameter of 0.55 mm, followed by the initial imidization processing and such a process was conducted nine times so that a thickness of the coating by the application at a time became 7 to 8 µm by varying the inner diameter of dies. Thus, the polyimide coating with a thickness of 85 µm was formed on the surface of the stainless-steel wire. After that, as the final imidization processing, heat was applied at 250°C for 10 minutes, followed by the increase of the temperature up to 400°C for 15 minutes and heating at the same temperature for 10 minutes, so as to complete the conversion to an imide. In this way, a polyimide coated stainless wire was obtained.

Next, a core wire only was elongated in the lengthwise direction by 0.1% or more, and the resin tube was pulled out, whereby a medical tube with an inner diameter of 0.55 mm and an outer diameter of 0.72 mm was manufactured. When the stainless-steel wire only was pulled out from the polyimide coated stainless-steel wire, it was cut into a predetermined length, and then the polyimide layer at the both end portions was removed. Then, the stainless-steel wire was elongated so as to decrease the outer diameter thereof, whereby the tube could be pulled out. According to the tube of the present invention, since the fluorine resin was precipitated on the inner face, the tube could be pulled out even in the case of a long tube.

The measurement results of the friction resistance of the inner face of this medical tube are shown in Table 1.

### (Comparative Example 1)

A polyimide coated stainless-steel wire was obtained in the same condition as in Example 1 except that fluorine resin powder was not mixed in the polyimide precursor solution. After that, a core wire only was pulled out, whereby a medical tube with an inner diameter of 0.55 mm and an outer diameter of 0.74 mm was manufactured.

The measurement results of the friction resistance of the inner face of this medical tube are shown in Table 1.

### (Example 2)

FEP powder with an average particle diameter of 14 µm (produced by Dupont, "532-8110" (Trade Name)) as a fluorine resin was added and mixed to the polyimide precursor solution made of BPDA/PPD prepared in Example 1 so that the ratio became 30 parts by weight of the FEP powder with respect to 100 parts by weight of solid content in the polyimide precursor solution, whereby a fluorine resin mixed polyimide precursor solution was prepared.

After that, similarly to Example 1, the polyimide precursor solution was applied to the stainless-steel wire with an outer diameter of 0.55 mm so that the coating with a thickness of about 85 µm was formed, and then the initial imidization processing was conducted similarly to Example 1. After that, as the final imidization processing, heat was applied at 250°C for 10 minutes, followed by the rise of temperature up to 300°C for 5 minutes and heating at 300°C for 15 minutes, whereby a polyimide coated stainless wire was obtained.

Next, a core wire only was pulled out, whereby a medical tube with an inner diameter of 0.55 mm and an outer diameter of 0.71 mm was manufactured. The measurement results of the friction resistance of the inner face of this medical tube are shown in Table 1.

### (Example 3)

The experiment was conducted similarly to Example 2 except that the final imidization temperature of Example 2 was changed at 350°C, whereby a polyimide coated stainless-steel wire was obtained. Next, a core wire only was pulled out, whereby a medical tube with an inner diameter of 0.55 mm and an outer diameter of 0.72 mm was manufactured. The measurement results of the friction resistance of the inner face of this medical tube are shown in Table 1.

### (Example 4)

Under the same conditions as those of the polyimide precursor solution made of (BPDA/PPD) prepared in Example 1, 75 parts by weight of ODA was dissolved in NMP with respect to 100 parts by weight of PMDA (monomer concentration: 18.0 weight%), whereby a polyimide precursor solution was prepared. The rotational viscosity of this polyimide precursor solution was 60 poise. Herein, the rotational viscosity was a value measured using a Brookfield viscometer at a temperature of 23°C. Next, PTFE powder with an average particle diameter of 3.0 µm (melting point: 327°C, produced by Dupont, "Zonyl MP1200" (Trade Name)) was added in the polyimide precursor solution so that the ratio became 30 parts by weight of the PTFE powder with respect to 100 parts by weight of solid content in the polyimide precursor solution, followed by stirring so as to disperse the powder uniformly. Subsequently, coarse foreign particles were filtered out using a stainless-steel wire netting of 250 mesh, whereby a fluorine resin powder mixed polyimide precursor solution was prepared.

After that, similarly to Example 1, the polyimide precursor solution was applied to the stainless-steel wire with an outer diameter of 0.55 mm so that the coating with a thickness of about 85 µm was formed, and then the initial imidization processing was conducted similarly to Example 1. After that, as the final imidization processing, heat was applied at 250°C for 10 minutes, followed by the rise of temperature up to 400°C for 15 minutes and heating at 400°C for 15 minutes, whereby a polyimide coated stainless wire was obtained.

Next, a core wire only was pulled out, whereby a medical tube with an inner diameter of 0.55 mm and an outer diameter of 0.71 mm was manufactured. The measurement results of the friction resistance of the inner face of this medical tube are shown in Table 1.

### (Example 5)

Similarly to Example 1, a polyimide precursor solution with a fluorine resin mixed therein was applied to the surface of a stainless-steel wire, and imidization processing was conducted thereto at 250°C. After that, a solution containing polyimide precursor alone made of BPDA/PPD prepared in Example 1 was applied to the outer face so that the coating with a thickness of 5 µm was formed, followed by drying. Then, similarly to Example 1, the temperature was increased up to 400°C for 15 minutes, followed by heating at the same temperature for 10 minutes, so as to complete the conversion to an imide, whereby a polyimide coated stainless-steel wire was obtained.

Next, a core wire only was pulled out, whereby a rigid medical tube with an inner diameter of 0.55 mm and an outer diameter of 0.73 mm was manufactured. The measurement results of the friction resistance of the inner face of this medical tube are shown in Table 1.

### (Comparative Example 2)

A polyimide coated stainless-steel was obtained under the same conditions as in Example 1 except that the final imidization temperature of Example 1 was changed to 300°C. Next, a core wire only was pulled out, whereby a medical tube with an inner diameter of 0.55 mm and an outer diameter of 0.72 mm was manufactured. The measurement results of the friction resistance of the inner face of this medical tube are shown in Table 1.

### (Reference Example 1)

The fluorine resin mixed polyimide precursor solutions prepared in the above Examples and Comparative examples were casting-molded on a glass plate of 300 mm□ (300 mm long and 300 mm wide) so that the thickness became 80 µm, and the conversion to an imide was completed at the final imidization temperatures of Table 1, whereby polyimide films were obtained. After that, the contact angle with pure water of each film was measured, and the measured results are shown in Table 1.

**(Table 1)**

| | Polyimide resin Acid component/ Amine component | Fluorine resin melting point (°C) | Final imidization temperature (°C) | Dynamic friction resistance (N) | Contact angle of film-form molded article (Ref. Example) | |
|---|---|---|---|---|---|---|
| | | | | | Glass-face contact angle (°) | Air-face contact angle (°) |
| Ex. 1 | BPDA/PPD | PTFE/327 | 400 | 0.18 | 106 | 108 |
| Comp. Ex. 1 | BPDA/PPD | - | 400 | 0.65 | 70 | 70 |
| Comp. Ex. 2 | BPDA/PPD | PTFE/327 | 300 | 0.60 | 75 | 72 |
| Ex.2 | BPDA/PPD | FEP/250 | 300 | 0.26 | 100 | 102 |
| Ex.3 | BPDA/PPD | FEP/250 | 350 | 0.23 | 96 | 101 |
| Ex.4 | PMDA/ODA | PTFE/327 | 400 | 0.19 | 105 | 108 |
| Ex. 5 | BPDA/PPD | PTFE/327 | 400 | 0.18 | 106 | 70 |

As is evident from Table 1, it can be confirmed that the tubes of the present invention have low dynamic friction resistance and a guide wire can be taken in and out smoothly. Further, from the measurement results of the contact angle of the film-formed molded articles, it can be confirmed that fluorine resin was precipitated on the surface of the polyimide resin tube. Thereby, it can be confirmed that repellency of the outer surface of the tube with blood can be enhanced, so that blood will not build up inside and the occurrence of a thrombosis will be prevented. A tube whose outer face was coated with a solution containing polyimide precursor alone had a large friction resistance of the outer face, and a contact angle thereof also was small. Therefore, even when it has a double-layered configuration with polyurethane or nylon on the outer face of the tube, the adhesion was favorable, and a composite medical tube with excellent rigidity of polyimide as well as flexibility and stability by other materials could be manufactured.

### Industrial Applicability

A tube of the present invention is applicable to a minute coated tube used for semiconductors and motors, as well as medical use.

## Claims

1. A medical tube comprising a mixture component including a polyimide resin and a fluorine resin, the mixture component being heated and cured,
wherein the fluorine resin melts and is precipitated on an inner face or the inner face and an outer face of the tube, and
the face on which the fluorine resin is precipitated is a low friction resistance face.

2. The medical tube according to claim 1, wherein a dynamic friction resistance of the inner face of the tube is 70% or less of that of a tube made of a polyimide resin alone.

3. The medical tube according to claim 1, wherein the content of the fluorine resin with reference to the polyimide resin is 3 to 50 weight%.

4. The medical tube according to claim 1, wherein the tube comprises a polyimide resin obtained by conversion to an imide by heating of a polyimide precursor solution including at least one type of aromatic tetracarboxylic acid dehydrate and at least one type of aromatic diamine.

5. The medical tube according to claim 1, wherein the fluorine resin is at least one selected from the group consisting of polytetrafluoroethylene (PTFE), tetrafluoroethylene-perfluoroalkylvinylether copolymer (PFA), polychlorotrifluoroethylene (PCTFE), tetrafluoroethylene-hexafluoropropylene copolymer (FEP) and tetrafluoroethylene-ethylene copolymer (PETFE).

6. The medical tube according to claim 1, wherein the medical tube is a catheter tube.

7. A method for manufacturing a medical tube, comprising the steps of:
polymerizing aromatic tetracarboxylic acid dehydrate and aromatic diamine in a polar solvent to be a polyimide precursor solution;
adding a fluorine resin in the polyimide precursor solution or during the polymerizing step to prepare a mixed solution of the polyimide precursor and the fluorine resin;
applying the mixed solution to an outer face of a core wire so as to have a predetermined thickness;
applying heat so as to allow conversion to an imide, where a highest temperature for the conversion to an imide is a temperature exceeding a melting point of the fluorine resin; and
thereafter, separating the core wire and the medical tube.

8. The method for manufacturing a medical tube according to claim 7, wherein before the conversion to an imide or after completion of the conversion to an imide, a solution containing a polyimide precursor alone is applied again, followed by conversion to an imide.

9. The method for manufacturing a medical tube according to claim 7, wherein the fluorine resin is at least one powder selected from the group consisting of polytetrafluoroethylene (PTFE), tetrafluoroethylene-perfluoroalkylvinylether copolymer (PFA), polychlorotrifluoroethylene (PCTFE), tetrafluoroethylene-hexafluoropropylene copolymer (FEP) and tetrafluoroethylene-ethylene copolymer (PETFE).

10. The method for manufacturing a medical tube according to claim 7, wherein an average particle diameter of the fluorine resin is 0.1 to 25 µm.
